# EUROPEAN PATENT SPECIFICATION

(11) **EP 0 491 930 B1**
(45) Date of publication and mention of the grant of the patent: **15.01.1997**
(21) Application number: 91913715.8
(22) Date of filing: 10.07.1991
(51) Int. Cl.: C12N 15/49, A61K 39/21, C12N 7/00

(54) **PRIMATE LENTIVIRUS VACCINES**
PRIMATEN-LENTIVIRUS IMPFSTOFFE
VACCINS PREPARES A PARTIR D'UN LENTIVIRUS DE PRIMATE

(30) Priority: 12.07.1990 US 551945
(43) Date of publication of application: 01.07.1992
(73) Proprietor: THE PRESIDENT AND FELLOWS OF HARVARD COLLEGE, Cambridge, MA 02138 (US)
(72) Inventor: DESROSIERS, Ronald, C., Hudson, MA 01749 (US)
(74) Representative: Deans, Michael John Percy
(86) International application number: US9104884
(87) International publication number: WO9200987

(56) References cited:
- PROCEEDINGS OF THE NATIONAL ACADEMY OF SCIENCES OF USA. vol. 84, March 1987, WASHINGTON US pages 1434 - 1438 LUCIW ET AL. 'Mutational analysis of the human immunodeficiency virus:The orf-B region down-regulates virus replication'
- SCIENCE. vol. 248, no. 4956, 1 June 1990, LANCASTER, PA US pages 1109 - 1112 KESTLER ET AL. 'Induction of AIDS in Rhesus Monkeys by molecularly cloned Simian Immunodeficiency Virus'
- SCIENCE. vol. 233, 8 August 1986, LANCASTER, PA US pages 655 - 659 FISHER ET AL. 'Infectious mutants of HTLV-II with changes in the 3'region and markedly reduced cythopatic effects'
- SCIENCE. vol. 258, no. 5090, 18 December 1992, LANCASTER, PA US pages 1880 - 1881 COHEN 'AIDS Vaccines:Is older better?'
- Science, Volume 233, issued 08 August 1986, FISHER et al, "Infectious mutants of HTLV-III with changes in the 3' region and markedly reduced cytopathic effects", pages 655-659. See entire article.
- AIDS Research and Human Retroviruses, Volume 6, Number 9, Issued September 1990, ZAGURY et al, "In vitro characterization of a biologically active molecular clone of HTV-2 NTH-2 containing a nef deletion and expressing a full-length transmembrane protein", pages 1079-1085. See entire article.
- Virology, Volume 173, Issued December 1989, HU et al, "Analysis of the function of viral protein X (VPX) of HIV-2", pages 624-630. See entire article.
- Journal of Virology, Volume 63, Number 9, issued September 1989, LU et al, "Effects of Long terminal repeat mutations on human immunodeficiency virus Type I replication", pages 4115-4119. See entire article.
- Proceedings of the National Academy of Sciences, Volume 87, Issued October 1990, HATTORI et al, "The human immunodeficiency virus type 2 vpr gene is essential for productive infection of human macrophages" pages 8080-8084. See entire acticle.
- Journal of Virology, Volume 64, Number 6, Issued June 1990, COHEN et al, "Human immunodeficiency virus vpr product is a virion-associated regulatory protein", pages 3097-3099. See entire article.
- The EMBO Journal, Volume 8, Number 4, Issued April 1989, GUYADER et al, "VPX mutants of HIV-2 are infectious in established cell lines but display a severe defect in peripheral blood lymphocytes", pages 1169-1175. See entire article.
- Proceedings of the National Academy of Sciences, USA, Volume 86, Issued July 1989, TERWILLIGER et al, "Functional role of human immuno deficiency virus type I Vpu", pages 5163-5167. See entire article.
- Journal of Virology, Volume 64, Number 10, Issued October 1990, LU et al., "Identification of cis-acting repressive sequences within the negative regulatory element of human immunodeficiency virus Type 1", pages 5226-5229. See entire Article.
- Science 258: 1938-1941 (Daniel et al. 1992)
- AIDS Res.Hum.Retroviruses 10: 221-222 (WHO working group 1994)
- Science 267: 633-636 (Paul 1995)
- N.Eng.J.Med. 332: 228-232 (Kirchhoff et al. 1995)
- Boston Business Journal, February 3-8, 1995, pages 1 and 31
- N.Eng.J.Med, 332, pages 259-260

## Description

This invention is in the general field of primate lentiviruses and their utilization as protective vaccines.

Human immunodeficiency virus type 1 (HIV-1), a member of the lentivirus subfamily of retroviruses, is the etiologic agent of the disease, acquired immune deficiency syndrome (AIDS) (F. Barre-Sinoussi et. al., *Science* 220:868, 1983, R. C. Gallo et. al., *Science* 224:500, 1984). This virus suppresses the immune system rendering the host highly susceptible to a variety of opportunistic infections and neoplasms. The AIDS epidemic is worldwide, with thousands of Europeans and possibly millions of Africans infected with the virus (T. C. Quinn et al., *Science* 234:955, 1986). As of December 1987, there were 47,000 cases of AIDS in the United States, and it was estimated that an additional 1 to 2 million individuals were infected but, at that time, asymptomatic (U.S. Public Health Service, *Public Health Rep.* 101:341, 1986). HIV infection is spread by sexual contact, by infected blood or blood products, and perinatally from mother to infant (A. S. Fauci et al., *Ann. Intern. Med.,* 100:92, 1984; J. W. Curran et al., *Science* 229:1352, 1985).

A closely related but distinct virus, human immunodeficiency virus type 2 (HIV-2) has been isolated from patients with a clinical syndrome comparable to HIV-1-induced AIDS. HIV-2 shares a significant degree of sequence homology and serologic reactivity with HIV-1 (F. Clavel, *N. Eng. J. of Med.* 316:1180, 1987).

B. Hahn et. al. *(Nature* 312:166, 1984) and G. M. Shaw et. al. *(Science* 226:1165, 1984) report the isolation of HIV-1 DNA clones capable of transmitting infection to cultured cells. L. Ratner et. al. *(Nature* 313:277, 1985,) and S. Wain-Hobson et. al. *(Cell* 40:9, 1985) report the complete nucleotide sequence of HIV-1.

The HIV-1 genome contains at least nine open reading frames. As catalogued by Cullen and W. C. Green *(Cell* 58:423, 1989), these encode structural proteins (Gag, Pol, Env), proteins required for virion morphogenesis and maturation (Vif, Vpu), nonstructural, regulatory proteins (Tat, Rev, Nef), and a protein of unknown function (Vpr).

Simian immunodeficiency viruses (SIVs) are nonhuman primate lentiviruses that are the closest known relatives of HIV-1 and HIV-2. They closely parallel their human counterparts in genetic organization and biological properties. Similarities between HIV and SIV include lentiviral morphology; tropism for CD4 lymphocytes and macrophages; extra genes called tat, rev, vif, ypf, and nef generally not present in other retroviruses; interaction with the CD4 receptor on host cells; cytopathicity; and the ability, of at least some, to cause chronic disease and death after long-term persistent infection (R.C. Desrosiers, *Annu. Rev. Microbiol.* 42:607, 1988).

SIV molecular clones have been isolated from macaque monkeys, although other species such as mangabeys may be their natural host, and are generally designated SIVmac (L. Chakrabarti et. al., *Nature* 328:543, 1987, V. Hirsch et. al., *Cell* 49:307, 1987, G. Franchini et. al., *Nature* 328:539, 1987). One clone, chosen for its ability to grow well in macaque peripheral blood lymphocytes, has been designated SIVmac239. (Y.M. Naidu et. al., *J. Virol.* 62:4691, 1988). Rhesus monkeys inoculated with stock virus derived from cloned SIVmac239 DNA develop an AIDS-like disease and eventually die (H. Kestler et. al., *Science* 248:1107, 1990).

To date, efforts at provoking a protective immune response against HIV have generally focused on specific antigens, such as HIV-encoded proteins. The sera of patients infected with HIV-1 contain antibodies that recognize the precursor and processed forms of the viral envelope (env) gene product, gp120 (J. S. Allan et. al., *Science* 228:1091, 1985, F. Barin et. al., *Science* 228:1094, 1985). These antibodies reportedly are not sufficient to impart immunity against AIDS, at least in the vast majority of infected individuals (J. Schupbach et. al., Science 224:503, 1984, M. Essex et. al., *Science* 220:859, 1983, Barre-Sinoussi et. al., Science 220:868, 1983). However, various Env-based vaccines are currently being studied (see, for example, D. Zagury et. al., *Nature* 332:728, 1988, W. C. Koff and A. S. Fauci, *AIDS 1989* 3 (supp1): S125, 1989).

A killed, non-infectious, envelope-depleted whole HIV-1 virus is also being tested in patients already infected with HIV-1 (A. Levine et. al., *Fifth International Conference on AIDS. The Scientific and Social Challenge.,* p. 219, 1989)

D. Baltimore *(Nature* 335:395, 1988) reports a proposed procedure for introducing, into a human subject, bone-marrow stem cells that are "infected or transfected with a virus or DNA construct that encodes an RNA or protein able dominantly to interfere with the intracellular growth of HIV, and propose that it be called intracellular immunization".

M. H. Malim et. al. *(Cell* 58:205, 1989) report the isolation of a Rev mutant that trans-dominantly inhibits wild-type Rev function and inhibits HIV-1 replication and say that trans-dominant Rev protein may be useful as a means of providing "intracellular immunization against HIV-1".

D. Trono et. al.(*Cell* 59:113, 1989) report that replication of wild-type HIV-1 virus is inhibited by co-expression of dominant negative Gag protein and say that these mutant proteins "appear to constitute suitable substrates for developing an antiviral scheme based on intracellular immunization".

M. Green et. al. (*Cell* 58:215, 1989) report that defective Tat peptides block trans-activation of the HIV-1 LTR and say that their "results suggest an attractive approach for the development of an AIDS therapy".

In one aspect, the invention features an infectious, non-pathogenic primate lentivirus, or a DNA clone of an infectious, non-pathogenic primate lentivirus, containing an engineered non-revertible null mutation in the nef gene.

We use the term "primate lentivirus genome" herein to mean the genetic material derived from a retrovirus, that, in wild-type form, (a) includes tat, rev, and nef genes, (b) is capable of infecting T4 cells of a primate host, and (c) possesses a viral morphogenesis and morphology characteristic of the lentivirus subfamily. The term includes, without limitation, all variants of HIV and SIV, including HTLV-III, ARV, LAV, HIV-1, HIV-2, SIVmac, SIVagm, SIVmnd, SIVsmm, SIVman, SIVmand, and SIVcpz. By "null mutation" is meant an alteration in the nucleotide sequence that renders the gene incapable of expressing a functional protein product. By "non-revertible" is meant unable to regain the ability to produce a functional protein product in the absence of wild-type virus or intentional genetic manipulation.

Preferred clones are derived from isolated primate lentiviruses; for example, they are produced by modifying a naturally occurring isolate to create the required nef mutation or by synthesizing nucleic acid to generally correspond to the sequence of a naturally occurring isolate, but with the required nef mutation. Thus, this invention is not limited to any specific viral clone. Those of ordinary skill in the art can readily isolate or engineer equivalent DNA clones which may differ in nucleic acid sequence by conservative substitutions such that the altered codon encodes a very similar amino acid, for example, a substitution of an alanine codon for a glycine codon. In addition, the nucleic acid sequence may have one or more non-conservative codon substitutions or one or more deletions which do not destroy the ability of the virus to produce progeny virus. Such equivalent DNA clones may be engineered by standard techniques of recombinant DNA technology, e.g., random or site-specific *in vitro* mutagenesis or deletion of sequences between restriction sites, and isolated by standard techniques of recombinant DNA technology including, but not limited to, the use of PCR and lambda, plasmid, cosmid, and/or other cloning vectors. Further, the invention is not limited to the specific non-revertible null mutation of the nef gene provided in the drawing; those skilled in the art can readily isolate other mutations which interfere with the ability of the nef gene to produce a functional protein product and which are non-revertible, e. g., larger or smaller deletions of the nef gene sequence. Such equivalent mutations may be engineered or isolated by standard techniques of recombinant DNA technology, e.g., by in vitro mutagenesis.

Primate lentiviruses (i.e., an RNA clone or virally packaged RNA) within the scope of the invention have a genome which contains an engineered (i.e., intentionally created or intentionally selected and isolated) non-revertible, null mutation in the nef gene.

Such a virus is infectious but not pathogenic, and it may be readily isolated by transfecting cultured primate cells with one of the DNA clones described above and harvesting progeny virus.

In preferred embodiment, the nucleic acid sequence of the DNA clone of the primate lentivirus is derived from a primate lentivirus, specifically HIV or SIV, particularly SIVmac, and the mutation leaves intact the env gene sequence.

In other preferred embodiments, the nucleic acid sequence of the DNA clone of the primate lentivirus includes, in addition to a nef mutation (as described above), a non-revertible null mutation in one or more of the following sequences: NRE, vpr, or vpx (in the case of a simian lentivirus) or vpu (in the case of a human lentivirus). Particularly preferred DNA clones according to the invention include SIVmac239ΔnefΔNRE, SIVmac239Δ3, SIVmac239Δ4, HIV-1ΔnefΔNRE, HIV-1Δ3, and HIV-1Δ4.

In other aspects, the invention features a vaccine comprises a virus or DNA clone in accordance with the first aspect of this invention in a pharmaceutically acceptable carrier.

Finally, the invention features a method of producing a vaccine by transfecting cultured primate cells with a primate lentiviral nucleic acid of a virus or DNA clone in accordance with the first aspect of the invention (i.e., one which contains an engineered non-revertible null mutation of the nef gene and which may also contain a non-revertible null mutation in the NRE, vpr, vpx, and/or vpu sequences), isolating lentivirus whose genome contains the mutation of the nef gene, and compounding the virus into a pharmaceutically acceptable vaccine.

Our vaccine provides immunological protection against primate lentiviruses using a live and infectious, but nonpathogenic, derivative of the parent virus. The introduction of non-revertible mutations provides an advantage over methods previously employed to produce attenuated viral vaccines, for example, isolation of viral stocks harboring genomic point mutation(s), capable of reverting to the wild-type sequence. In addition, construction of the engineered viral DNA clone is rapid, and the method of producing the vaccine by propagation of attenuated virus in mammalian cell culture is both rapid and inexpensive.

Other features and advantages of the invention will be apparent from the following description of preferred embodiments.

The drawings will first be described.

Fig. 1 is a representation of the complete nucleic acid and amino acid sequence of the SIVmac239 genome, one example of a primate lentivirus useful in practice of this invention.

The boundaries of the nef open reading frame and the extent of the nef deletion in the mutant clone, PSIVmac239 nef-deletion, are shown.

Fig. 2 is a representation of the complete nucleic acid and amino acid sequence of an isolate of HIV-1. The nef open reading frame is denoted E', and its boundaries are shown.

Fig. 3 (SEQ ID NO:5) is a representation of the complete nucleic acid sequence of HIV NL43.

### Primate lentivirus clones

There now follow examples of DNA clones according to the invention which include a deletion in the nef gene of SIVmac239 or HIV-1. All nef gene sequences isolated to date exhibit homology at the nucleotide and amino acid levels *(Human Retroviruses and AIDS 1990. A Compilation and Analysis of Nucleic Acid and Amino Acid Sequences,* ed. G. Myers et. al., Los Alamos National Laboratory, Los Alamos, NM). In addition, the position of the nef gene in the lentiviral genome (i.e., between the env gene and the 3' LTR) is conserved *(Human Retroviruses and AIDS 1990. A Compilation and Analysis of Nucleic Acid and Amino Acid Sequences,* ed. G. Myers et. al., Los Alamos National Laboratory, Los Alamos, NM). It is therefore a matter within the skill in the art to identify the nef gene sequence in any known or newly isolated primate lentivirus. Using standard techniques, such as those described below, said nef gene can be appropriately mutated, tested for pathogenicity, and compounded into a suitable vaccine that evokes protection against the AIDS virus.

The following clones are described to illustrate, not to limit, the invention. As described below, those of ordinary skill in the art can readily produce DNA clones from other SIV or HIV isolates having similar utility in the production of a nonpathogenic viral vaccine against AIDS.

### SIVmac239 nef-deletion

Parental virus SIVmac239 was isolated from a macaque monkey (New England Regional Primate Research Center) that exhibited impaired T-cell function, lymphoproliferative disorders, and opportunistic infections, symptoms characteristic of SIV infection. Cell free serum samples from this animal were cocultivated with HuT-78 cells (a human tumor T-cell line available from American Type Culture Collection, 12301 Parklawn Drive, Rockville, MD 20852 under accession No. TIB 161) and infectious SIVmac239 virus isolated from the HuT-78 cell supernatant as described in M. D. Daniel et al. *(Science* 228:1201, 1985). The cell supernatant was shown to possess detectable reverse transcriptase activity (by the method of M. D. Daniel et. al., *Science* 228:1201, 1985) beginning 12 to 18 days after initiation of cocultivation. The presence of viral particles in this cell line was confirmed by electron microscopy; SIVmac239 infected cells displayed budding viral particles with a morphology characteristic of primate lentiviruses.

To facilitate cloning of the full length viral genome, endonuclease EcoRI was identified as a noncutter of SIVmac239 DNA. Total cell DNA was prepared from SIVmac239-infected HuT-78 cells and digested with EcoRI. Sucrose density gradient size-fractionated 10- to 20-kilobase pair EcoRI fragments were inserted into the EcoRI site of lambda cloning vector, EMBL4. This library was screened using pK2 BamA (described in V. Hirsch et. al., *Cell* 49:307, 1987) as a probe, and full length molecular clone, SIVmac239, was isolated. The nucleotide sequence of both strands was determined by the primer-directed dideoxy-chain termination method of Sanger et. al. *(Proc. Natl . Acad. Sci .* USA 74:5463, 1977) using Sequenase (i.e., T7 DNA polymerase) versions 1.0 or 2.0 (United States Biochemical, Cleveland, OH). ³⁵S-labelled sequencing reactions were electrophoresed on a buffer gradient (0.75 to 2.5% Tris-borate buffer) 6% polyacrylamide/8M urea gel. Sequences were entered into an IBM-PC computer with an IBI gel reader and digitizer and were analyzed with IBI-Pustell DNA analysis software (International Biotechnologies, Inc., New Haven, CT).

The SIVmac239 clone in EMBL4 was then used to generate two plasmid subclones as follows. EMBL-SIVmac239 was digested with SphI, and a 6706 base pair fragment, containing the sequence from the SphI site in the left flanking cellular DNA sequence to the SphI site at viral nucleotide number 6451, was inserted into the SphI site of vector pBS(+) (Stratagene, La Jolla, CA) to produce subclone, p239SpSp5'. In a separate reaction, EMBL-SIVmac239 was digested with SphI and EcoRI, and a 6361 base pair fragment, containing the sequence from the SphI site at viral nucleotide 6452 to the EcoRI site in the right flanking cellular sequence, was inserted into SphI/EcoRI cleaved vector pBS(-) (Stratagene, La Jolla, CA) to produce subclone, p239SpE3'. These subclones were used to generate a full length genomic sequence as follows. Both p239SpSp5'and p239SpE3' were digested with SphI, ligated together, and used to directly transfect cultured cells (e.g., macaque peripheral blood lymphocytes).

A non-revertible null mutation (a deletion) was engineered into the nef gene of the SIVmac239 clone as follows. The C-terminus of the nef gene, contained within an SstI fragment of p239SpE3' and encompassing nucleotides 9230 to 11,436, was cloned into M13 to produce M13-SSTB. This M13 clone was subjected to oligonucleotide site specific deletion mutagenesis using procedures described in M. J. Zoller and M. Smith *(DNA* 3:479, 1984) or M. Strauss et. al. *(Gene* 49:331, 1986). A 73 base pair oligonucleotide complementary to bases (9215 through 9250) and (9433 through 9469) was synthesized. The two segments were contiguous, i.e., bases 9251 through 9432 were not present on the 73-mer. This oligonucleotide was annealed to single stranded M13-SSTB in the presence of Klenow polymerase and the four deoxyribonucleotides to promote synthesis of the complementary strand. Since bases 9251 through 9432 were not included in the oligonucleotide primer, their complement was similarly not included in the newly synthesized negative strand. Double stranded replicative form (RF) phage DNA was transformed into E. coli host, JM109. DNA from the resulting M13 plaques was immobilized on nitrocellulose filters by standard techniques (W. D. Benton and R. W. Davis, *Science* 196:180, 1977) and screened for the sequence in the deleted region, using an oligonucleotide probe complementary to the deleted sequence. A negative signal was taken as an indication that a clone contained a nef deletion. One M13 clone, confirmed to have the expected deletion by DNA sequencing, was subsequently used for reconstruction of the full length viral sequence as follows. The fragment, containing the deletion, was liberated by digestion of deleted M13-SSTB, with SstI. Said fragment was then cloned into p239SpE3' to yield p239SpE3'(nef-deletion). Plasmid subclones, p239SpSp5' and p239SpE3'(nef-deletion), were digested with SphI, ligated together, and used to directly transfect cultured cells (e.g., macaque peripheral blood lymphocytes).

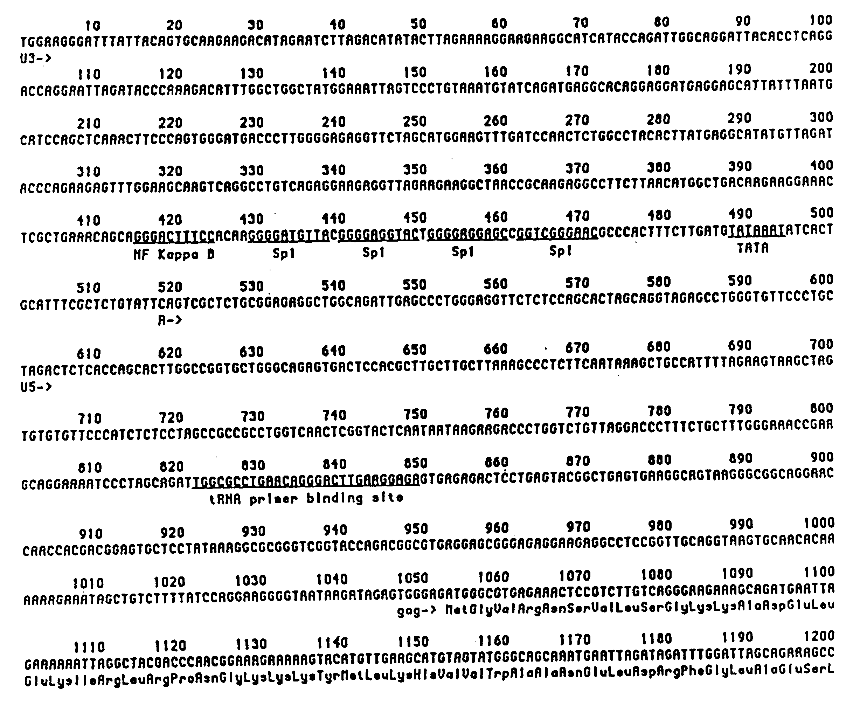

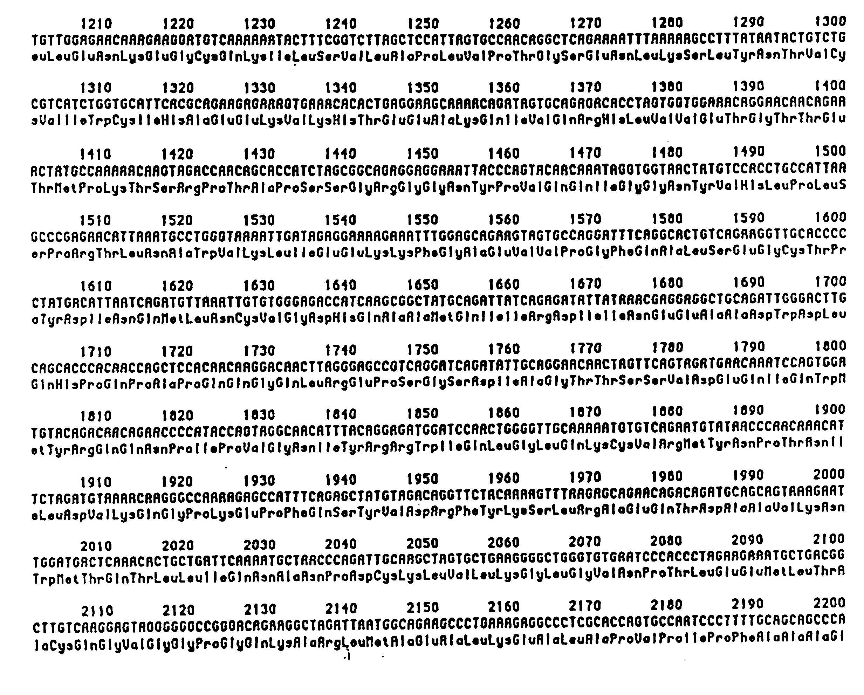

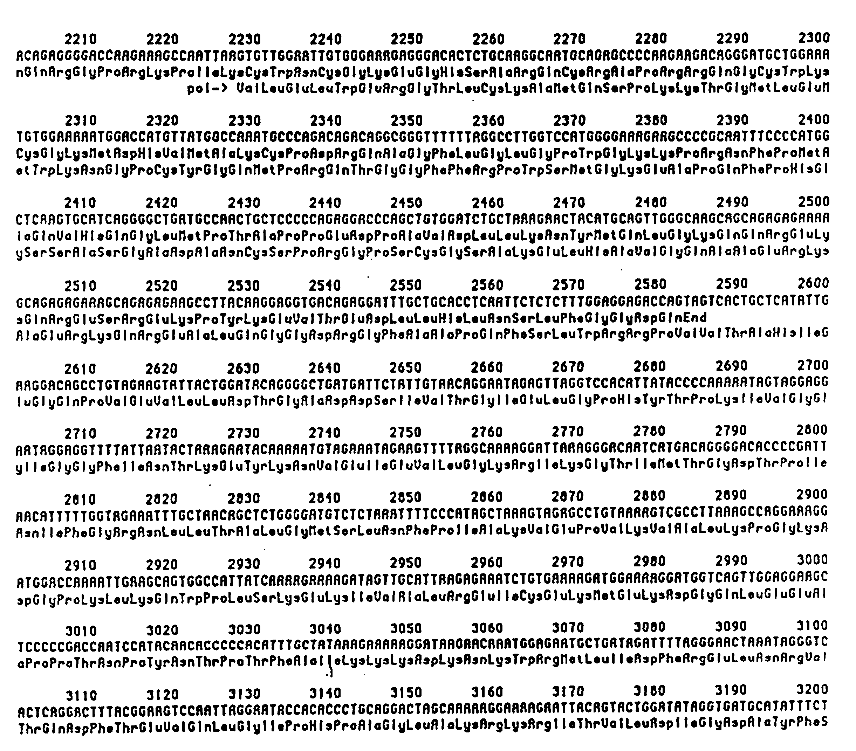

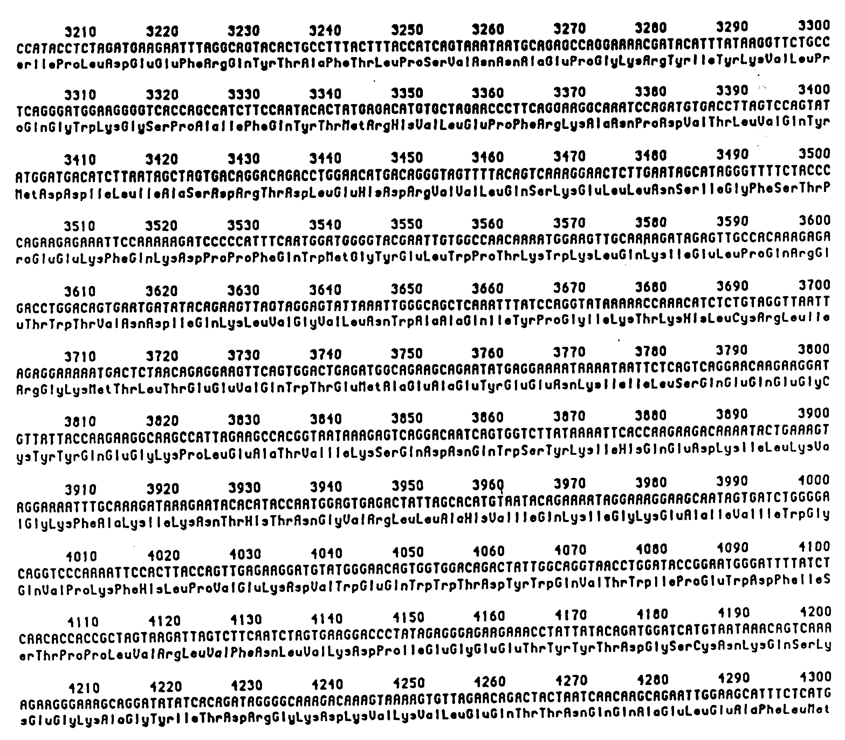

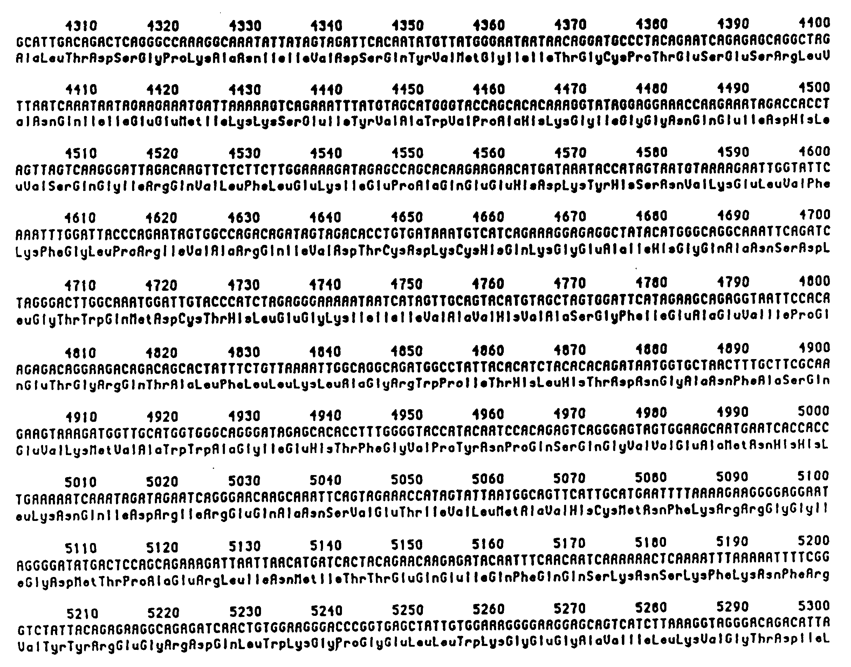

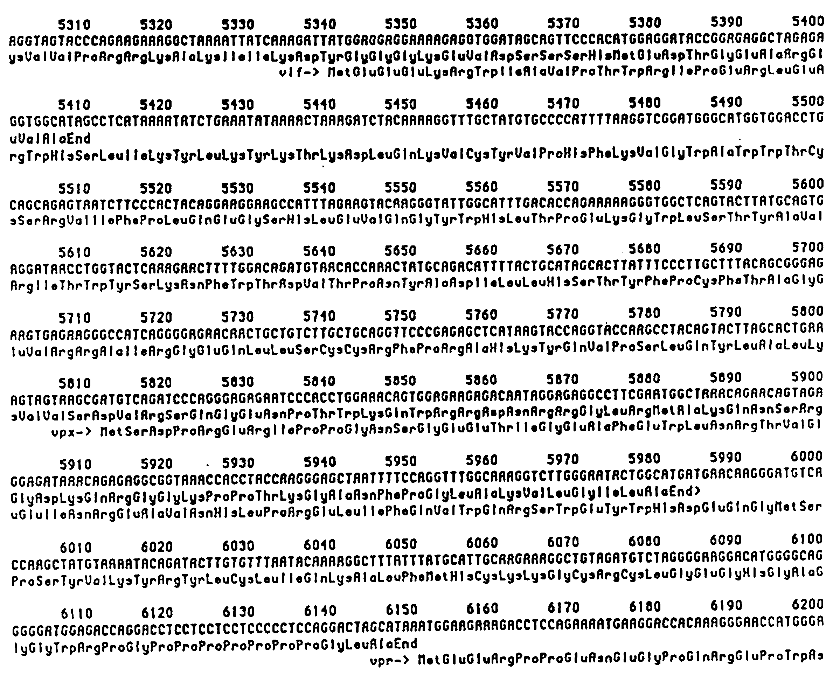

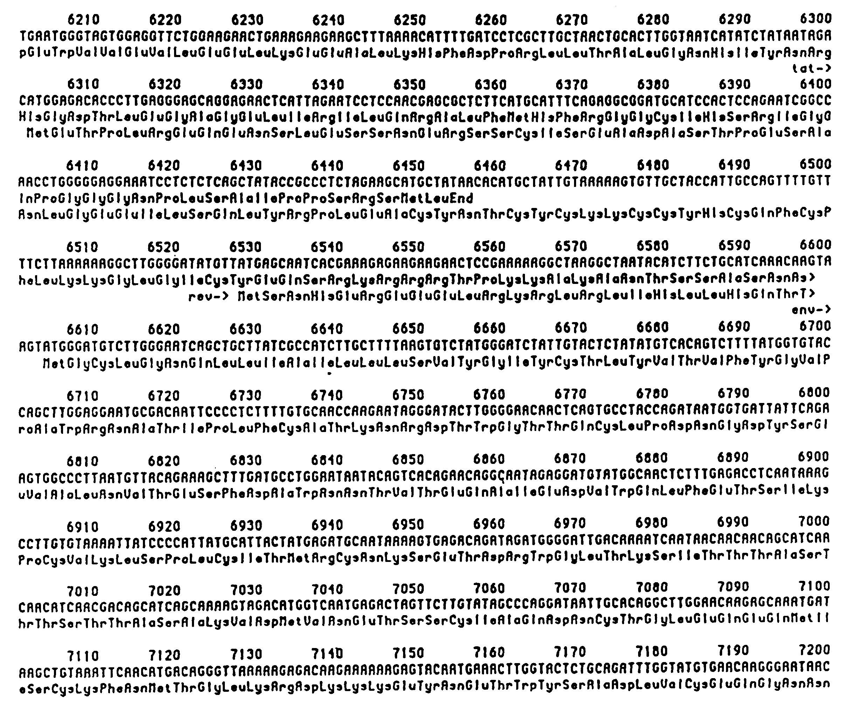

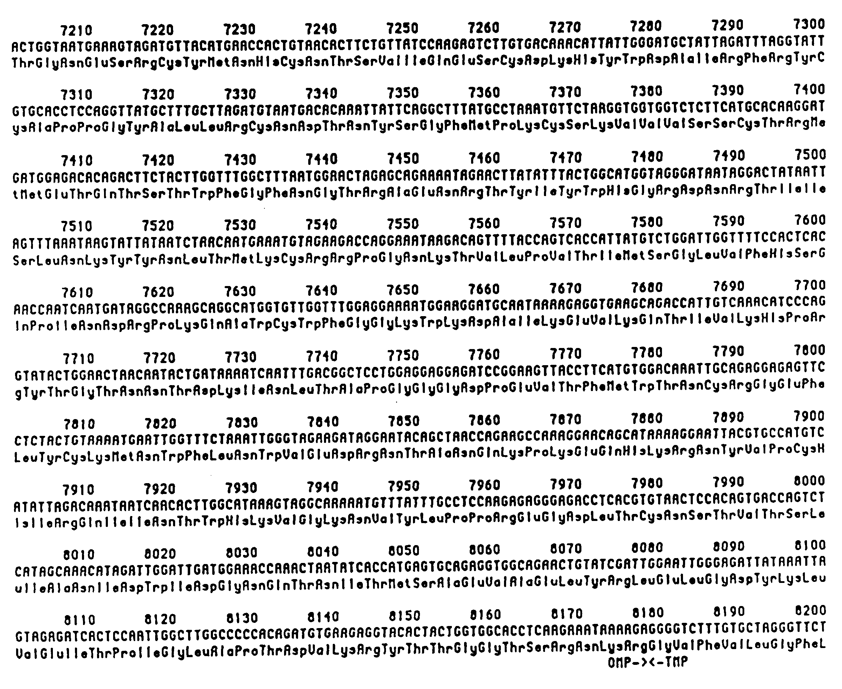

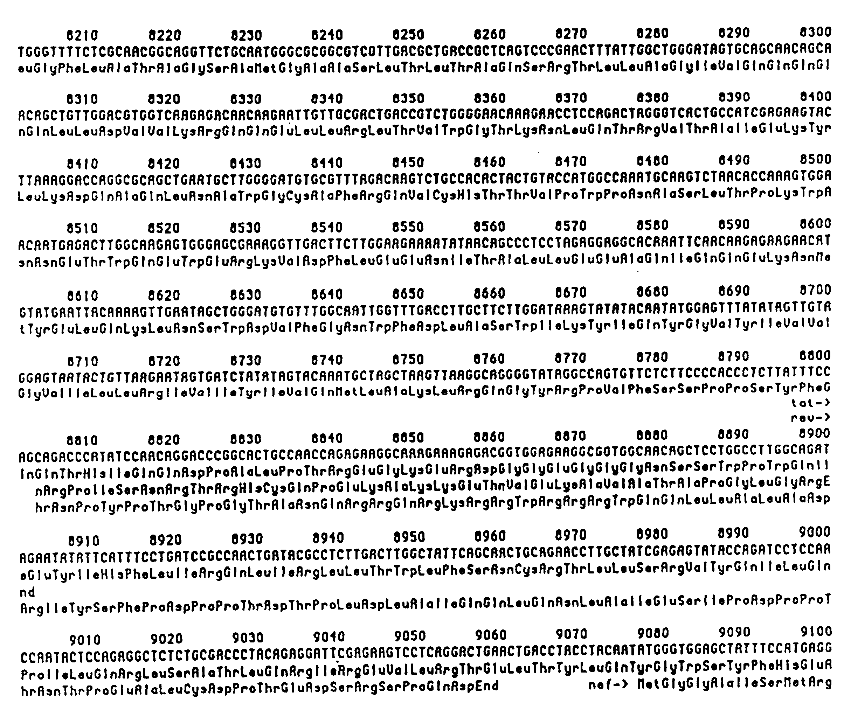

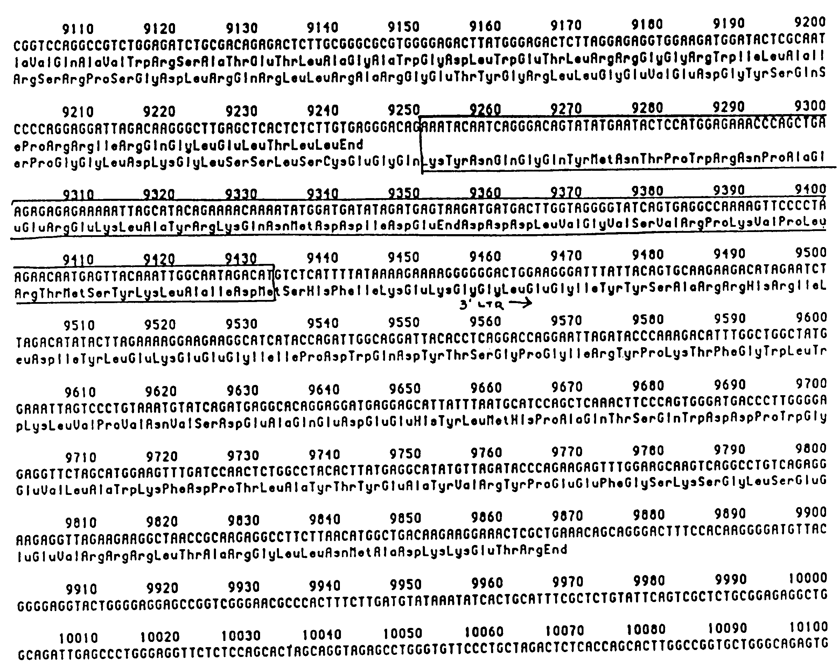

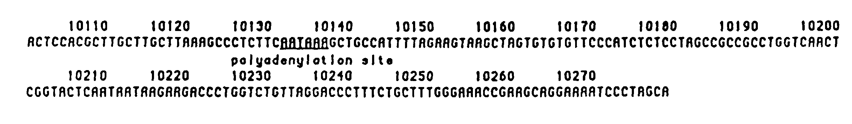

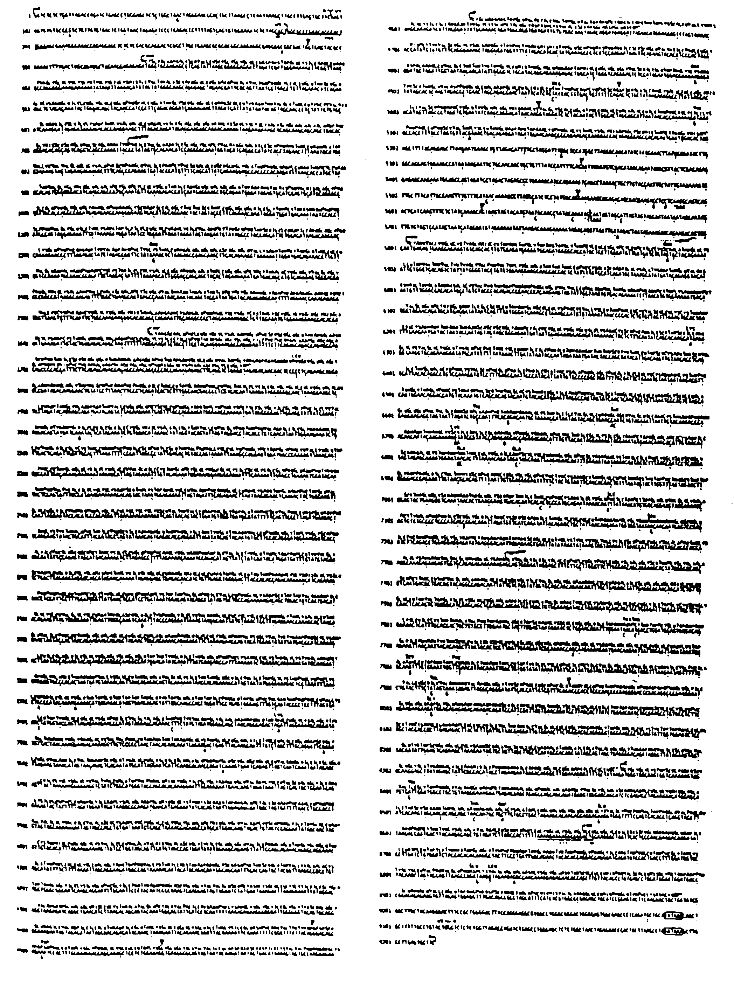

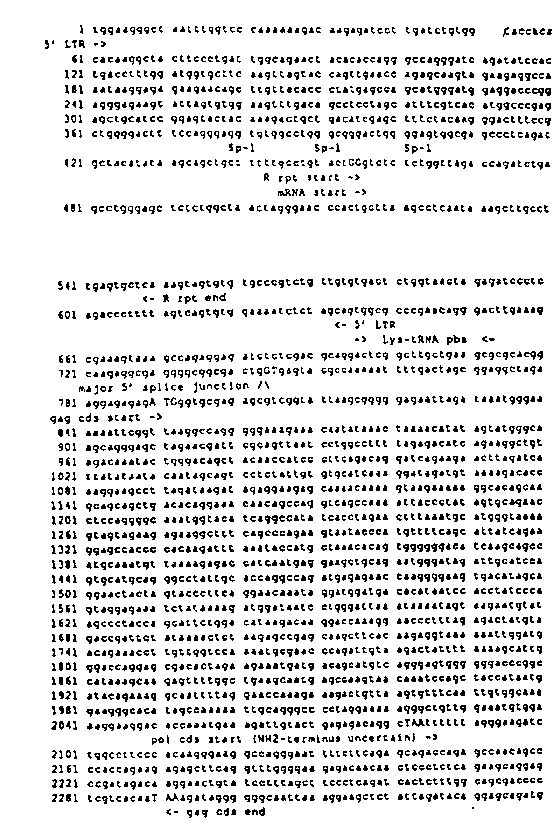

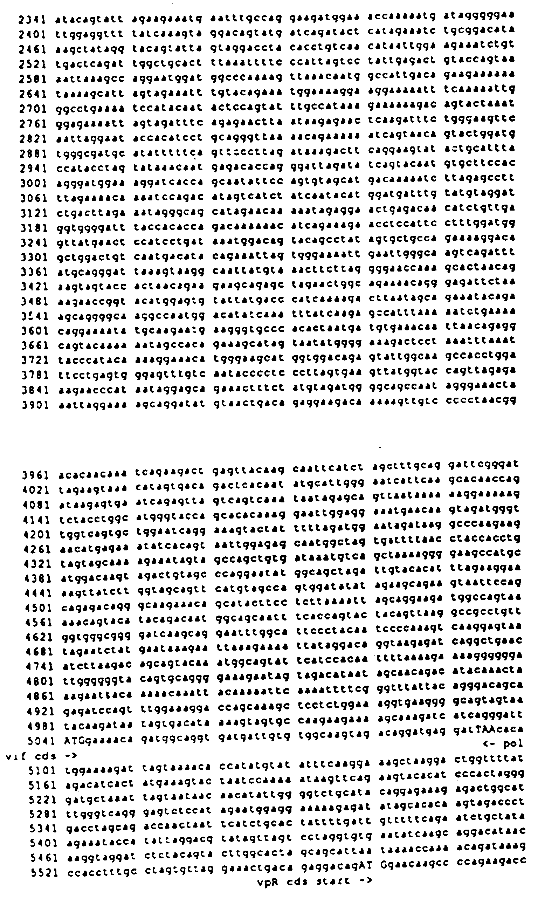

As shown in Figure 1, the SIVmac239 nef gene overlaps the env gene sequence. Disruption of the env gene sequence of the SIVmac239 genome, or, generally, any PLV genome, can render the lentivirus non-infectious and, hence, not useful for the production of an effective live vaccine. For this reason, the nef deletion, contained in p239SpE3'(nef-deletion), leaves the env gene intact.

Plasmid clones p239SpSp5' and p239SpE3'(nef-deletion) have been deposited with the American Type Culture Collection and they, respectively, bear the accession numbers: ATCC No. 68365 and No. 68364.

Using the SIVmac239 sequence provided in Figure 1, one skilled in the art can engineer other non-revertible null mutations in the nef gene sequence. Deletions that remove a portion (≥ 10%) of the SIV nef open reading frame can be easily verified by restriction mapping. Such deletions of the Nef encoding nucleic acid, or deletions that disrupt Nef control or response sequences, in most cases, render the gene incapable of producing a Nef gene product. The absence of Nef gene product can be verified by immunoassay (e.g., Western blot analysis), using purified antibodies directed to the Nef protein or serum from primates infected with a primate lentivirus. When other types of null mutations are accomplished (e.g., smaller deletions), their efficacy can be tested using an *in vivo* assay. Briefly, for a simian immunodeficiency virus, an aliquot of purified virus derived from a mutant nef clone can be injected intravenously into a live, permissive host, preferably a rhesus monkey, and tested for pathogenicity (i.e., ability to induce AIDS-like disease symptoms). Virus harboring a genome with a null mutation in the nef gene (e.g., SIVmac239 nef-deletion) are nonpathogenic.

### SIVmac239ΔnefΔNRE

Another example of an SIV DNA clone according to the invention, i.e., one which includes a deletion in-the nef gene and a deletion in the NRE sequence of the lonq terminal repeat (LTR), was constructed as follows.

Plasmid p239SpE3'(nef-deletion) was digested with NsiI and StuI, enzymes which cut at unique sites within the NRE region of the SIVmac LTR. The DNA fragment comprising most of the plasmid sequence (but lacking the internal NRE fragment) was isolated, the protruding ends blunt-ended using T4 DNA polymerase, and the blunt ends ligated together to recreate a circular plasmid. The plasmid was re-isolated, and was confirmed, by nucleotide sequence analysis, to be lacking nucleotides 9660 to 9831 (in the NRE region); this plasmid was termed p239SpE3'ΔnefΔNRE.

Plasmids p239SpSp5' and p239SpE3'ΔnefΔNRE were each digested with SphI, ligated together, and used to directly transfect permissive cultured cells (e.g., macaque peripheral blood lymphocytes) as described above. Such cells produced an SIVmac239 virus whose genome included a deletion in the nef gene and a deletion in the NRE sequence (i.e., SIV mac239ΔnefΔNRE).

### SIVmac239Δ3

Another example of an SIV DNA clone according to the invention, i.e., one which includes deletions in the nef and vpr genes and in the NRE sequence was constructed as follows.

Plasmid p239SpSp5' was subjected to polymerase chain reaction (PCR) site specific mutagenesis by the method of Ho et al. (*Gene* 77:51, 1989). 35 bp oligonucleotides, complementary to bases (6135 through 6152) and (6254 through 6270) of SIVmac239, respectively, were synthesized and used as the PCR primers. Specifically such oligonucleotides were of sequence: 
5' TCCAGGACTAGCATAAATTTGATCCTCGCTTGCTA 3' (SEQ ID
NO:1) and
 
5' TAGCAAGCGAGGATCAAATTTATGCTAGTCCTGGA 3'(SEQ ID
NO:2).
 The resultant plasmid, termed p239SpSp5'Δvpr, included a deletion of nucleotides 6153 through 6253 of the vpr gene.

Plasmids p239SpSp5'Δvpr and p239SpE3'ΔnefΔNRE were each digested with SphI, ligated together, and used to directly transfect permissive cultured cells (e.g., macaque peripheral blood lymphocytes) as described above. Such cells produced an SIVmac239 virus whose genome included deletions in the nef and vpr genes and in the NRE sequence (i.e., SIVmac239Δ3).

### SIVmac239Δ4

Another example of an SIV DNA clone according to the invention, i.e., one which includes a deletion in the nef, vpr, and vpx genes and in the NRE sequence was constructed as follows.

Plasmid p239SpSp5' was subjected to PCR-site specific mutagenesis as described above. 30 bp oligonucleotides, complementary to bases (5973 through 5987) and (6254 through 6268) of SIVmac239, respectively, were synthesized and used as PCR primers. Specifically, such oligonucleotides were of sequence: 
5' ATACTGGCATGATGATTGATCCTCGCTTGC 3'(SEQ ID NO:3)
and
 
5' GCAAGCGAGGATCAATCATCATGCCAGTAT 3' (SEQ ID NO:
4).
 The resultant plasmid, termed p239SpSp5'ΔvpxΔvpr, included a deletion of nucleotides 5988 through 6253 of the vpx and vpr genes.

Plasmids p239SpSp5'ΔvpxΔvpr and p239SpE3'ΔnefΔNRE were each digested with SphI, ligated together, and used to directly transfect permissive cultured cells (e.g., macaque peripheral blood lymphocytes) as described above. Such cells produced an SIVmac239 virus whose genome included deletions in the nef, vpr, and vpx genes and in the NRE sequence (i.e., SIVmac239Δ4).

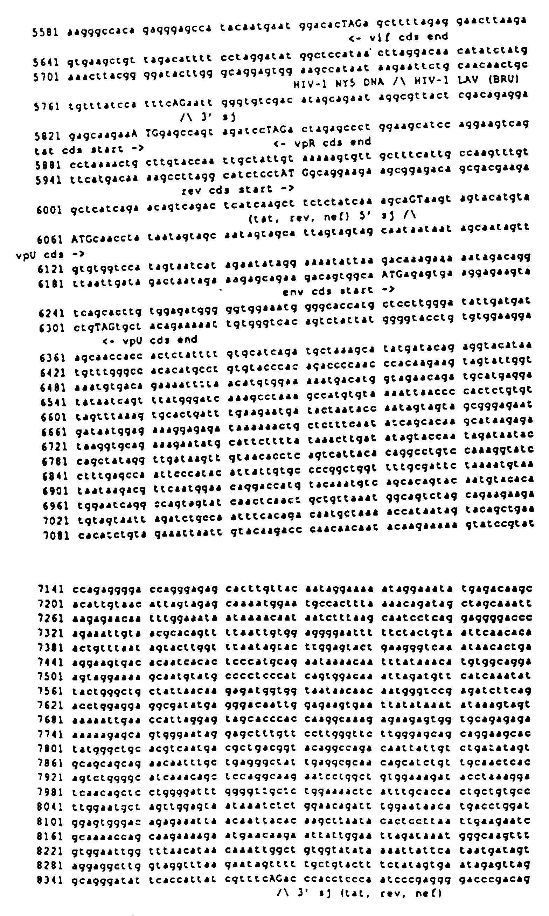

Non-revertible null mutations can also be engineered into the genomic nef sequence of other primate lentivirus genomes, for example, HIV-1. Figure 2 shows the complete nucleotide and amino acid sequence of an isolate of HIV-1; as indicated in this figure, the nef gene (referred to as E') includes the sequence between nucleotides 8,347 and 8,992. Said sequence is located between the env gene and the 3' LTR and exhibits nucleotide and amino acid sequence homology to the nef gene of the simian lentivirus isolate, SIVmac239. Non-revertible null mutations can be introduced into the HIV-1 nef sequence by standard recombinant DNA techniques, for example, site directed *in vitro* mutagenesis or deletion of sequence between restriction sites. Again, deletions that remove all or most of the HIV-1 nef open reading frame can be verified by restriction mapping or by immunoassay. Deletions which disrupt Nef control or response elements, in most cases, also render the gene incapable of producing a Nef gene product, and this event also could be tested by immunoassay as described above for SIV Nef. When other types of null mutations (e.g., smaller deletions) are accomplished, their efficacy should be tested using an *in vivo* assay. No permissive host (except humans) exists for directly testing the pathogenicity of a mutant HIV clone *in vivo.* However, as described below, due to the similarity in the human and simian nef sequences, an equivalent SIV nef-mutant clone (i.e., with a deletion in the corresponding SIV Nef domain) can be engineered and can be tested for pathogenicity in rhesus monkeys as described above.

There now follow specific examples of HIV-1 clones according to the invention. These examples are for the purpose of illustrating, not limiting, the invention.

### HIV-1ΔnefΔNRE

An example of an HIV DNA clone according to the invention, i.e., one which includes a deletion in the nef gene and in the NRE sequence is constructed as follows.

Beginning with the proviral clone, termed pNL4-3 (Adachi et al., *J. Virol.* 59:284, 1986; available from the AIDS Research and Reference Reagent Program, NIAID, National Institute of Health, Bethesda, MD), the HIV-1 sequence is sub-cloned into two separate vectors. Specifically, pNL4-3 is digested with ApaLI and EcoRI and a 5897 bp fragment including bp -154 (within the flanking cellular sequence) to bp 5743 (within the flanking cellular sequence) (i.e., the viral genes, gag, pol, vif, and vpr) is inserted into a plasmid vector, termed pDR8, to produce pLEFT.

Plasmid pDR8 was constructed by digesting pUC19 with NdeI and EarI (i.e., deleting the lacZ and lacI genes and the polylinker) and rendering the protruding ends blunt with T4 DNA polymerase. Into this backbone was inserted, by blunt-end ligation, an oligonucleotide of sequence:

5' TGGTGACCTTCGAAGGATCCCATATGTCTAGAGAATTCGGTCACCA 3'
3' ACCACTGGAAGCTTCCTAGGGTATACAGATCTCTTAAGCCAGTGGT 5' (SEQ
ID NO:14), to produce pDR8.

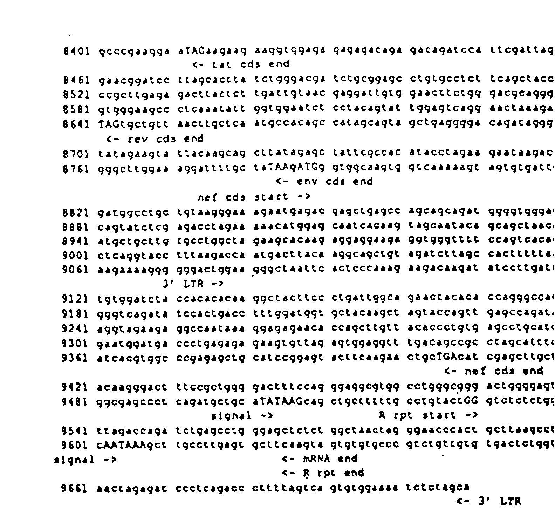

In a separate reaction pNL4-3 is digested with EcoRI and ThaI and a 4002 bp fragment including bp 5743 of Fig. 3 (SEQ ID NO:5) to bp 9745 (in the flanking cellular sequence) (i.e., the viral genes, tat, rev, vpu, env, nef, and LTR) is inserted into plasmid vector, pDR8, to produce pRIGHT.

To mutate the nef and NRE genes, pRIGHT is digested with NdeI or ApaLI and ThaI and at 3247 bp or 3136 bp viral fragment, respectively, is isolated and inserted into plasmid vector pDR8, to create pRIGHT-SUB1. The nef gene and NRE sequence are mutated by PCR-site specific mutagenesis by the method of Ho et al. (supra). PCR primers are chosen such that most or all of the nef and NRE sequences are deleted. For example, deletion of the nef gene may be accomplished using PCR primers of sequence: 
5' AAGGATTTTGCTATAATAGCCACTTTTTTAAAA 3' (SEQ ID
NO:6) and
 
5' TTTTAAAAAGTGGCTTATTATAGCAAAATCCTT 3'(SEQ ID
NO: 7),
 complementary to bases (8770 through 8785) and (9047 through 9062) of the HIV-1 sequence of Fig. 3 (SEQ ID NO:5).

Deletion of the NRE sequence may be accomplished using PCR primers of sequence: 
5' ACTGACCTTTGGATGGCATCCGGAGTACTTCA (SEQ ID NO:8)
and
 
5' TGAAGTACTCCGGATGCCATCCAAAGGTCAGT 3' (SEQ ID
NO:9),
 complementary to bases (9194 through 9209) and (9381 through 9396) of the HIV-1 sequence of Fig. 3 (SEQ ID NO:5). The mutated version of pRIGHT-SUB1 is digested with NdeI or ApaLI (corresponding to the enzyme chosen above) and ThaI and the 3075 bp or 2964 bp fragment, respectively isolated and re-inserted into NdeI- or ApaLI- and ThaI-digested pRIGHT to create pRIGHT-MUT1. pRIGHT-MUT1 and pLEFT are digested with EcoRI, ligated together, and used to directly transfect permissive cultured cells (e.g., human peripheral blood lymphocytes) as described in Sompayrac and Danna *(Proc. Natl. Acad. Sci*. *USA* 78:7575, 1981) or Milman and Herzberg *(Somat. Cell Genet.* 7:161, 1981) or Naidu et al. *(J. Virol.* 62:4691, 1988). Such cells produce an HIV-1 virus whose genome includes a deletion in the nef gene and in the NRE sequence (i.e., HIV-1ΔnefΔNRE).

### HIV-1Δ3

Another example of an HIV DNA clone according to the invention, i.e., one which includes deletions in the nef and vpr genes and in the NRE sequence is constructed as follows.

Plasmids pRIGHT-MUT1 and pLEFT are constructed as described above. A fragment containing the vpr gene is further subcloned. Specifically, pLEFT is digested with BspMI or NdeI and EcoRI and a 697 bp or 621 bp viral fragment, respectively, is isolated and inserted into plasmid vector, pDR8, to create pLEFT-SUB1. The vpr gene is mutated by PCR-site specific mutagenesis (by the method of Ho et al., supra). PCR primers are designed such that most or all of the vpr gene is deleted. For example, primers may be of sequence: 
5' AATGAATGGACACTAGTAATAAGAATTC 3' (SEQ ID NO:10)
and
 5' GAATTCTTATTACTAGTGTCCATTCATT 3' (SEQ ID NO:11), complementary to bases (5604 through 5619) and (5737 through 5748) of the HIV-1 sequence of Fig. 3 (SEQ ID NO:5). The mutated version of pLEFT-SUB1 is digested with BspMI or NdeI (corresponding to the enzyme chosen above) and EcoRI, and the 579 bp or 503 bp viral fragment, respectively, isolated and re-inserted into BspMI- or NdeI- and EcoRI-digested pLEFT to create pLEFT-MUT. pLEFT-MUT and pRIGHT-MUT1 are digested with EcoRI, ligated together, and used to directly transfect permissive cultured cells (e.g., human peripheral blood lymphocytes) as described in Sompayrac and Danna *(Proc. Natl. Acad. Sci. USA* 78:7575, 1981) or Milman and Herzberg *(Somat. Cell Genet.* 7:161, 1981) or Naidu et al. *(J. Virol.* 62:4691, 1988). Such cells produce an HIV-1 virus whose genome includes deletions in the nef and vpr genes and in the NRE sequence (i.e., HIV-1Δ3).

### HIV-1Δ4

Another example of an HIV DNA clone according to the invention, i.e., one which includes deletions in the nef, vpr, and vpu genes and in the NRE sequence is constructed as follows.

Plasmids pLEFT-MUT, pRIGHT, and pRIGHT-MUT1 are constructed as described above.

A fragment containing the vpu gene is subcloned. Specifically, pRIGHT is digested with EcoRI and NdeI or ApaLI and a 864 bp or 656 bp fragment, respectively, is isolated and inserted into plasmid vector, pDR8, to create pRIGHT-SUB2. The vpu gene is mutated by PCR-site specific mutagenesis by the method of Ho et al. (supra). PCR primers are chosen such that most or all of the vpu gene is deleted. For example, primers may be of sequence: 
5' GTAAGTAGTACATGTAATGAGAGTGAAGGAGA 3' (SEQ ID
NO:12) and
 
5' TCTCCTTCACTCTCATTACATGTACTACTTAC 3' (SEQ ID
NO:13),
 complementary to bases (6045 through 6060) and (6221 through 6236) of the HIV-1 sequence of Fig. 3 (SEQ ID NO:5). The mutated version of pRIGHT-SUB2 is digested with EcoRI and NdeI or ApaLI (corresponding to the enzyme chosen above) and the 475 bp or 686 bp (respectively) viral fragment isolated and inserted into an EcoRI- and NdeI- or ApaLI-digested pRIGHT-MUT1 backbone, to create pRIGHT-MUT2. pRIGHT-MUT2 and pLEFT-MUT are digested with EcoRI, ligated together, and used to directly transfect permissive cultured cells (e.g., human peripheral blood lymphocytes) as described in Sompayrac and Danna *(Proc. Natl. Acad. Sci. USA* 78:7575, 1981) or Milman and Herzberg *(Somat. Cell Genet.* 7:161, 1981) or Naidu et al. *(J. Virol.* 62:4691, 1988). Such cells produce an HIV-1 virus whose genome includes deletions in the nef, vpr, and vpu genes and in the NRE sequence (i.e., HIV1Δ4).

### Isolation of Primate Lentivirus

Having produced an infectious DNA clone containing a nef mutation according to the invention, that clone is used to transfect appropriate cells, for example, immortalized lymphocytes, such as H9 cells or HuT-78 cells, or peripheral blood lymphocytes from donor mammals. After culturing the infected cells, primate lentiviruses, suitable for making a vaccine, can be isolated from the cell supernatant following centrifugation and filtration, using procedures identical to those utilized to isolate wild-type virus.

In order to more fully illustrate the manner of isolating primate lentivirus, the following example is presented.

### Isolation of SIVmac239 nef-deletion Virus

SIVmac239 nef-deletion virus can be isolated by digesting p239SpSp5' and p239SpE3'(nef-deletion) DNA with SphI, ligating together, and transfecting into HuT-78 cells by the DEAE-dextran technique of Sompayrac and Danna *(Proc. Natl. Acad. Sci. USA* 78:7575, 1981) and Milman and Herzberg *(Somatic Cell Genet.* 7:161, 1981) followed by isolation of progeny virus as follows.

Five aliquots of viral DNA (3 ug of ligated DNA/aliquot) are sequentially added, with intermittent mixing, to 1.4 ml of serum-free Dulbecco's modified Eagle medium (GIBCO, Grand Island, N.Y.) containing 125 ug/ml DEAE-dextran and 50 mM Tris (pH 7.3). HuT-78 cells are split 1:2 or 1:3, grown to 3 x 10⁶ cells/plate (i.e., 24 hours or less), washed twice with Dulbecco's modified Eagle medium, mixed with the 1.4 ml DNA solution, and incubated at 37°C for 1 hr. The cells are then washed with serum-free Dulbecco modified Eagle medium, washed with serum-free RPMI 1640 medium (GIBCO, Grand Island, NY), and incubated at 37°C in RPMI 1640 medium containing 10% fetal calf serum. Transfected cells are split 1:2 or 1:3, twice per week.

Alternatively, SIVmac239 nef-deletion may be propagated by transfecting H9 cells by the above procedure or by transfecting macaque peripheral blood lymphocytes essentially by the above procedure, except that the cells are stimulated with 1 ug/ml phytohemagglutinin (GIBCO, Grand Island, NY) for 48 hrs prior to transfection and grown in RPMI 1640 medium containing 10% interleukin-2 (lectin-free, T-cell growth factor; Electro-Nucleonics, Inc., Fairfield, N.J.) following transfection.

Generally, seven to fourteen days after transfection, cells are removed by centrifugation (1500g, 10 minutes, 4°C), and the supernatant is filtered through a disposable 0.45 micron filter (Corning, Corning, NY). Cell-free supernatant is monitored for reverse transcriptase activity, using the methods of M. D. Daniel et. al. *(Science* 228:1201, 1985), or for the amount of Gag antigen, by antigen capture (Coulter Immunology, Hialeah, FL). Cell free supernatant, possessing detectable reverse transcriptase activity and viral antigen, is saved as virus stock. Stocks may be stored in liquid nitrogen for long periods of time, e.g., one to two years, without significant loss of virus viability. A cycle of freezing and thawing, however, may reduce viral infectivity approximately ten-fold.

### Vaccine Production

A PLV molecular clone containing a non-revertible null mutation in the nef gene or a virus which harbors such a mutant genome is used to transfect or infect appropriate primate cells, e.g., immortalized HuT-78 or H9 cells, or peripneral blood lymphocytes, using procedures described above. After a standard incubation time, for example, seven to fourteen days, the culture medium is removed by aspiration, the cells are washed twice with phosphate-buffered saline (137mM NaCl, 2.7mM KCI, 4.3mM Na₂HPO₄^{.}7H₂O, 1.4mM KH₂PO₄, pH 7.3) and the medium is replaced with serum-free RPMI 1640. Following an appropriate incubation time in serum-free medium (approximately 12 to 24 hours), virus is harvested by centrifugation and further purified by passage of the cell free supernatant through a 0.45 micron filter as described above. Viral stock is titered by standard techniques *(Techniques in HIV Research,* ed. A. Aldovini and B. D. Walker, Stockton Press, New York, NY), diluted, if necessary, in a pharmaceutically acceptable carrier, such as physiological saline, and stored frozen in aliquots appropriate for a single immunizing dose. Stabilizers (e.g., MgCl₂, hydrolyzed gelatin, or sorbitol), antibiotics (e.g., streptomycin or neomycin), and pH indicators (e.g., phenol red), may also be added to the vaccine just prior to storage.

### Method of Vaccination

An immunizing amount (i.e., an infecting dose) of this vaccine is administered by any standard procedure, for example, by intramuscular injection. This dose is effective over the course of at least twelve months.

Other embodiments are within the following claims. For example, the genome may be substantially identical to a PLV genome, but for the nef mutation discussed above and a non-revertible null mutation in one or more of the other PLV genes that are not essential to infectivity, for example, vpr, vpu, or vif (e.g., as described above), or the deletion of LTR sequences that are not essential for virus replication.

## Claims

1. An infectious, non-pathogenic primate lentivirus, or a DNA clone of an infectious, non-pathogenic primate lentivirus, containing an engineered non-revertible null mutation in the nef gene.

2. A virus or DNA clone according to Claim 1, wherein said mutation leaves intact the genomic env gene sequence.

3. A virus or DNA clone according to Claim 1, wherein said clone is derived from the genome of a human immunodeficiency virus, preferably the genome of HIV-1.

4. A virus or DNA clone according to Claim 1, wherein said clone is derived from the genome of a simian immunodeficiency virus, preferably the genome of SIVmac.

5. A virus or DNA clone according to Claim 4, comprising the DNA clones deposited with the ATCC and designated No. 68364 and No. 68365.

6. A virus or DNA clone according to Claim 1, further comprising an engineered non-revertible null mutation in the NRE sequence.

7. A virus or DNA clone according to Claim 1, further comprising an engineered non-revertible null mutation in the vpr gene sequence.

8. A virus or DNA clone according to Claim 1, further comprising an engineered non-revertible null mutation in the vpx gene sequence.

9. A virus or DNA clone according to Claim 1, further comprising an engineered non-revertible null mutation in the vpu gene sequence.

10. A virus or DNA clone according Claim 1, wherein said virus or clone is derived from SIV and comprises a non-revertible null mutation in the nef gene and in the NRE gene.

11. A virus or DNA clone according to Claim 10, wherein said virus or clone lacks nucleotides 9251 to 9432 and 9660 to 9831 of SIVmac239.

12. A virus or DNA clone according to Claim 1, wherein said virus or clone is derived from SIV and comprises a non-revertible null mutation in the nef gene, in the NRE gene and in the vpr gene.

13. A virus or DNA clone according to Claim 12, wherein said virus or clone lacks nucleotides 9251 to 9432, 9660 to 9831 and 6153 to 6253 of SIVmac239.

14. A virus or DNA clone according to Claim 1, wherein said virus or clone is derived from SIV and comprises a non-revertible null mutation in the nef gene, in the NRE gene, in the vpr gene and in the vpx gene.

15. A virus or DNA clone according to Claim 14, wherein said virus or clone lacks nucleotides 9251 to 9432, 9660 to 9831, and 5988 to 6253 of SIVmac239.

16. A virus or DNA clone according to Claim 1, wherein said virus or clone is derived from HIV-1 and comprises a non-revertible null mutation in the nef gene and in the NRE gene.

17. A virus or DNA clone according to Claim 16, wherein said virus or clone lacks nucleotides 8786 to 9046 and 9210-9380 of pNL4-3.

18. A virus or DNA clone according to Claim 1, wherein said virus or clone is derived from HIV-1 and comprises a non-revertible null mutation in the nef gene, in the NRE gene and in the vpr gene.

19. A virus or DNA clone according to Claim 18, wherein said virus or clone lacks nucleotides 8786 to 9046, 9210 to 9380 and 5620 to 5736 of pNL4-3.

20. A virus of DNA clone according to Claim 1, wherein said virus or clone is derived from HIV-1 and comprises a non-revertible null mutation in the nef gene, in the NRE gene, in the vpr gene and in the vpu gene.

21. A virus or DNA clone according to Claim 20, wherein said virus or clone lacks nucleotides 8786 to 9046, 9210 to 9380, 5620 to 5736 and 6061 to 6220 of pNL4-3.

22. A method of producing a vaccine that provides protection against a primate lentivirus comprising the steps of: transfecting cultured primate cells with primate lentiviral nucleic acid of the virus or DNA clone according to any one of Claims 1 to 21; isolating lentivirus whose genome contains said mutation of the nef gene; and compounding said virus into a pharmaceutically acceptable vaccine.

23. A vaccine comprising a virus or DNA clone according to any of Claims 1 to 21 in a pharmaceutically acceptable carrier.

24. A virus or DNA clone according to any of Claims 1 to 21, for use in vaccination.

25. Use of a virus or DNA clone according to any of Claims 1 to 21 for the manufacture of a vaccine.

## Patentansprüche

1. Infektiöser, nicht pathogener Primaten-Lentivirus oder DNA-Klon eines infektiösen, nicht pathogenen Primaten-Lentivirus, der eine gentechnologisch veränderte, nicht rückmutierende Nullmutation im nef-Gen enthält.

2. Virus oder DNA-Klon nach Anspruch 1, bei dem die Mutation die genomische env Gensequenz intakt läßt.

3. Virus oder DNA-Klon nach Anspruch 1, bei dem der Klon von dem Genom eines menschlichen Immunschwächevirus, bevorzugt dem Genom von HIV-1, abstammt.

4. Virus oder DNA-Klon nach Anspruch 1, bei dem der Klon von dem Genom eines Affen-Immunschwächevirus, bevorzugt dem Genom von SIVmac, abstammt.

5. Virus oder DNA-Klon nach Anspruch 4, aufweisend die DNA-Klone, die bei der ATCC hinterlegt sind und mit den Nummern 68364 und 68365 bezeichnet sind.

6. Virus oder DNA-Klon nach Anspruch 1, außerdem aufweisend eine gentechnologisch veränderte, nicht rückmutierende Nullmutation in der NRE-Sequenz.

7. Virus oder DNA-Klon nach Anspruch 1, außerdem aufweisend eine gentechnologisch veränderte, nicht rückmutierende Nullmutation in der vpr-Gensequenz.

8. Virus oder DNA-Klon nach Anspruch 1, außerdem aufweisend eine gentechnologisch veränderte, nicht rückmutierende Nullmutation in der vpx-Gensequenz.

9. Virus oder DNA-Klon nach Anspruch 1, außerdem aufweisend eine gentechnologisch veränderte, nicht rückmutierende Nullmutation in der vpu-Gensequenz.

10. Virus oder DNA-Klon nach Anspruch 1, bei dem der Virus oder Klon von SIV abstammt und eine nicht rückmutierende Nullmutation in dem nef-Gen und in dem NRE-Gen enthält.

11. Virus oder DNA-Klon nach Anspruch 10, bei dem dem Virus oder Klon die Nucleotide 9251 bis 9432 und 9660 bis 9831 von SIVmac239 fehlen.

12. Virus oder DNA-Klon nach Anspruch 1, bei dem der Virus oder Klon von SIV abstammt und eine nicht rückmutierende Nullmutation in dem nef-Gen, in dem NRE-Gen und in dem vpr-Gen aufweist.

13. Virus oder DNA-Klon nach Anspruch 12, bei dem dem Virus oder Klon die Nucleotide 9251 bis 9432, 9660 bis 9831 und 6153 bis 6253 von SIVmac239 fehlen.

14. Virus oder DNA-Klon nach Anspruch 1, beim der der Virus oder Klon von SIV abstammt und eine nicht rückmutierende Nullmutation in dem nef-Gen, in dem NRE-Gen, in dem vpr-Gen und in dem vpx-Gen aufweist.

15. Virus oder DNA-Klon nach Anspruch 14, bei dem dem Virus oder Klon die Nucleotide 9251 bis 9432, 9660 bis 9831 und 5988 bis 6253 von SIVmac239 fehlen.

16. Virus oder DNA-Klon nach Anspruch 1, bei dem der Virus oder Klon von HIV-1 abstammt und eine nicht rückmutierende Nullmutation in dem nef-Gen und in dem NRE-Gen aufweist.

17. Virus oder DNA-Klon nach Anspruch 16, bei dem dem Virus oder Klon die Nucleotide 8786 bis 9046 und 9210 bis 9380 von pNL4-3 fehlen.

18. Virus oder DNA-Klon nach Anspruch 1, bei dem der Virus oder Klon von HIV-1 abstammt und eine nicht rückmutierende Nullmutation in dem nef-Gen, in dem NRE-Gen und in dem vpr-Gen aufweist.

19. Virus oder DNA-Klon nach Anspruch 18, bei dem dem Virus oder Klon die Nucleotide 8786 bis 9046, 9210 bis 9380 und 5620 bis 5736 von pNL4-3 fehlen.

20. Virus oder DNA-Klon nach Anspruch 1, bei dem der Virus oder Klon von HIV-1 abstammt und eine nicht rückmutierende Nullmutation in dem nef-Gen, in dem NRE-Gen, in dem vpr-Gen und in dem vpu-Gen aufweist.

21. Virus oder DNA-Klon nach Anspruch 20, bei dem dem Virus oder Klon die Nucleotide 8786 bis 9046, 9210 bis 9380, 5620 bis 5736 und 6061 bis 6220 von pNL4-3 fehlen.

22. Verfahren zum Erzeugen eines Impfstoffes, der Schutz gegen einen Primaten-Lentivirus schafft, folgende Schritte aufweisend:
Transfizieren gezüchteter Primatenzellen mit Primaten-Lentivirus-Nucleinsäure des Viruses oder DNA-Klons nach einem der Ansprüche 1 bis 21; Isolieren von Lentivirus, dessen Genom die Mutation des nef-Gens enthält; und Mischen des Viruses in einen pharmazeutisch annehmbaren Impfstoff.

23. Impfstoff, aufweisend einen Virus oder DNA-Klon nach einem der Ansprüche 1 bis 21 in einem pharmazeutisch annehmbaren Träger.

24. Virus oder DNA-Klon nach einem der Ansprüche 1 bis 21 zur Verwendung bei Impfungen.

25. Verwendung eines Viruses oder DNA-Klons nach einem der Ansprüche 1 bis 21 für die Herstellung eines Impfstoffs.

## Revendications

1. Lentivirus de primate non-pathogène, infectieux ou clone d'ADN d'un lentivirus de primate non-pathogène, infectieux, contenant une mutation nulle non-réversible introduite dans le gène nef.

2. Virus ou clone d'ADN selon la revendication 1, dans lequel ladite mutation laisse intact la séquence génomique du gène env.

3. Virus ou clone d'ADN selon la revendication 1, dans lequel ledit clone est dérivé du génome d'un virus de l'immunodéficience humaine, de préférence du génome d'HIV-1.

4. Virus ou clone d'ADN selon la revendication 1, dans lequel ledit clone est dérivé du génome d'un virus de l'immunodéficience du singe, de préférence du génome de SlVmac.

5. Virus ou clone d'ADN selon la revendication 4, comprenant les clones d'ADN déposés ayant les Numéros ATCC 68364 et 68365.

6. Virus ou clone d'ADN selon la revendication 1, comprenant en outre une mutation nulle non-réversible introduite dans la séquence NRE.

7. Virus ou clone d'ADN selon la revendication 1, comprenant en outre une mutation nulle non-réversible introduite dans la séquence du gène vpr.

8. Virus ou clone d'ADN selon la revendication 1, comprenant en outre une mutation nulle non-réversible introduite dans la séquence du gène vpx.

9. Virus ou clone d'ADN selon la revendication 1, comprenant en outre une mutation nulle non-réversible introduite dans la séquence du gène vpu.

10. Virus ou clone d'ADN selon la revendication 1, dans lequel ledit virus ou clone est dérivé de SIV et comprend une mutation nulle non-réversible dans le gène nef et dans le gène NRE.

11. Virus ou clone d'ADN selon la revendication 10, dans lequel il manque audit virus ou clone les nucléotides 9251 à 9432 et 9660 à 9831 de SlVmac239.

12. Virus ou clone d'ADN selon la revendication 1, dans lequel ledit virus ou clone est dérivé de SIV et comprend une mutation nulle non-réversible dans le gène nef, dans le gène NRE ainsi que dans le gène vpr.

13. Virus ou clone d'ADN selon la revendication 12, dans lequel il manque audit virus ou clone les nucléotides 9251 à 9432, 9660 à 9831 et 6153 à 6253 de SlVmac239.

14. Virus ou clone d'ADN selon la revendication 1, dans lequel ledit virus ou clone est dérivé de SIV et comprend une mutation nulle non-réversible dans le gène nef, dans le gène NRE, dans le gène vpr et dans le gène vpx.

15. Virus ou clone d'ADN selon la revendication 14, dans lequel il manque audit virus ou clone les nucléotides 9251 à 9432, 9660 à 9831 et 5988 à 6253 de SlVmac239.

16. Virus ou clone d'ADN selon la revendication 1, dans lequel ledit virus ou clone est dérivé de HIV-1 et comprend une mutation nulle non-réversible dans le gène nef et dans le gène NRE.

17. Virus ou clone d'ADN selon la revendication 16, dans lequel il manque audit virus ou clone les nucléotides 8786 à 9046 et 9210 à 9380 de pNL4-3.

18. Virus ou clone d'ADN selon la revendication 1, dans lequel ledit virus ou clone est dérivé de HIV-1 et comprend une mutation nulle non-réversible dans le gène nef, dans le gène NRE ainsi que dans le gène vpr.

19. Virus ou clone d'ADN selon la revendication 18, dans lequel il manque audit virus ou clone les nucléotides 8786 à 9046, 9210 à 9380 et 5620 à 5736 de pNL4-3.

20. Virus ou clone d'ADN selon la revendication 1, dans lequel ledit virus ou clone est dérivé de HIV-1 et comprend une mutation nulle non-réversible dans le gène nef, dans le gène NRE, dans le gène vpr ainsi que dans le gène vpu.

21. Virus ou clone d'ADN selon la revendication 18, dans lequel il manque audit virus ou clone les nucléotides 8786 à 9046, 9210 à 9380, 5620 à 5736 et 6061 à 6220 de pNL4-3.

22. Procédé de préparation d'un vaccin qui procure une protection vis à vis d'un lentivirus de primate comprenant les étapes consistant à transfecter les cellules de primate en culture avec l'acide nucléique de lentivirus de primate du virus ou du clone d'ADN selon l'une quelconque des revendications 1 à 21, à isoler le lentivirus dont le génome contient ladite mutation du gène nef et à conditionner ledit virus dans un vaccin acceptable d'un point de vue pharmaceutique.

23. Vaccin comprenant un virus ou un clone d'ADN selon l'une quelconque des revendications 1 à 21 dans un véhicule acceptable d'un point de vue pharmaceutique.

24. Virus ou clone d'ADN selon l'une quelconque des revendications 1 à 21, utile à la vaccination.

25. Utilisation d'un virus ou d'un clone d'ADN selon l'une quelconque des revendications 1 à 21 pour la préparation d'un vaccin.
